# EUROPÄISCHE PATENTSCHRIFT

(11) **EP 4 121 149 B1**
(45) Veröffentlichungstag und Bekanntmachung des Hinweises auf die Patenterteilung: **21.08.2024**
(21) Anmeldenummer: 21712488.2
(22) Anmeldetag: 15.03.2021
(51) Int. Cl.: A24F 40/50, A61M 15/06, A61M 11/04

(54) **VERFAHREN ZUM BESTIMMEN MINDESTENS EINES PARAMETERS FÜR DIE VERDAMPFUNG IN EINEM INHALATOR, UND INHALATOR**
METHOD FOR DETERMINING AT LEAST ONE PARAMETER FOR THE VAPORISATION IN AN INHALER, AND INHALER
PROCÉDÉ DE DÉTERMINATION D'AU MOINS UN PARAMÈTRE DE VAPORISATION DANS UN INHALATEUR, ET INHALATEUR

(30) Priorität: 16.03.2020 DE 102020107091
(43) Veröffentlichungstag der Anmeldung: 25.01.2023
(73) Patentinhaber: Körber Technologies GmbH, 21033 Hamburg (DE)
(72) Erfinder: GOLDSCHMIDTBÖING, Frank, 77799 Ortenberg (DE); PELZ, Uwe, 79227 Schallstadt (DE); GHANAM, Muhannad, 79114 Freiburg (DE); JAMALI, Armin, 79110 Freiburg (DE); BÄUMKER, Eiko, 79106 Freiburg (DE); SABERI, Mohammadreza, 79108 Freiburg (DE); WOIAS, Peter, 79100 Freiburg (DE); BILGER, Thomas, 79426 Buggingen (DE); JAKLIN, Jan, 70736 Fellbach (DE)
(74) Vertreter: Müller Verweyen
(86) Internationale Anmeldenummer: PCT/EP2021/056495
(87) Internationale Veröffentlichungsnummer: WO 2021/185740

(56) Entgegenhaltungen:
- WO-A1-2015/192084

## Beschreibung

Die vorliegende Erfindung betrifft ein Verfahren zum Bestimmen mindestens eines Parameters für die Verdampfung in einem Inhalator umfassend einen auf Widerstandsheizung beruhenden Verdampfer und eine elektronische Steuerungsvorrichtung.

Die Temperatur am Verdampfer wird typischerweise mithilfe eines temperaturabhängigen elektrischen Widerstands des Verdampfers ermittelt. Durch die Beziehung zwischen Temperatur und dem elektrischen Widerstand des Verdampfers kann die Temperatur des Verdampfers gezielt eingestellt werden. Die Temperatur sollte dabei eine durch die zu verdampfende Flüssigkeit bestimmte Temperatur nicht übersteigen, da sonst Schadstoffe entstehen können, insbesondere durch ein Trockenfallen des Verdampfers.

Der Stromkreis eines Verdampfers bzw. Heizers lässt sich vereinfacht als eine Reihenschaltung von elektrischen Widerständen beschreiben. Elemente dieser Reihenschaltung umfassen einen elektrischen Widerstand des Verdampfers (Verdampferwiderstand), einen Batterieinnenwiderstand sowie unerwünschte parasitäre elektrische Widerstände. Die parasitären Widerstände sind beispielsweise durch folgende Widerstände gegeben: einen zu der elektrischen Steuerungseinrichtung gehörigen elektrischen Widerstand, einen Strommesswiderstand, einen elektrischen Widerstand der Zuleitungen, insbesondere durch Verbindungsdrähte, Kupferleitbahnen und/oder Lötstellen und gegebenenfalls einen elektrischen Widerstand einer möglichen Steckverbindung. Der parasitäre Widerstand ist weder zeitlich konstant noch reproduzierbar, da beispielsweise Steckverbindungen je nach Alterungszustand, Verschmutzung und/oder Verformung einen nur mit erheblichem Aufwand messbaren Einfluss auf den parasitären Widerstand haben.

Temperaturmessfehler aufgrund parasitärer Widerstände können zu einer Überhitzung der zu verdampfende Flüssigkeit führen, was zu Blasensieden oder Schadstoffentstehung führen kann. Aufgrund der vielfältigen, durch Messung und parasitäre Ströme bedingten Fehler kann der Verdampfer nur unzureichend mit bekannten Verfahren geregelt werden.

Eine Temperaturmessung durch die Widerstandsänderung des Heizelements bzw. Verdampfers ist unter folgenden Umständen sehr ungenau: wenn eine Verdampferkartusche über einen lösbaren Verbinder mit einem Basisteil des Inhalators verbunden ist; wenn das Heizelement bzw. der Verdampfer einen geringen Widerstand im Ohm-Bereich und/oder einen geringen Temperaturkoeffizienten aufweist. Dies ist vor allem auf die Variation des Widerstands des Verbinders aufgrund variierender Anpresskraft, Verschmutzung, ggf. Korrosion, zurückzuführen.

Wie bereits in der deutschen Patentanmeldung Nr. 10 2019 113 645.8 beschrieben, ist es ist für die Verdampferregelung im normalen Konsumbetrieb vorteilhaft, den Widerstand, bzw. bei bekannter Spannung den Strom zu messen, bei dem die Verdampfung einsetzt (Übergangspunkt). Dies erfolgt vorteilhaft online während der regulären Verdampfung und somit in Echtzeit. Die Erkennung des Verdampfungspunkts (Übergangspunkts) anhand des zeitlichen Verlaufs des Widerstands bzw. Stroms ist schwierig, insbesondere wenn sie in Echtzeit in dem Inhalator erfolgen soll.

WO 2015/192084 A1 offenbart ein Verfahren zur Bestimmung des Spulenwiderstands und der Änderungsrate des Spulenwiderstands eines Inhalators sowie den Widerstand des Heizelements am Siedepunkt der verdampften Flüssigkeit.

Es ist die Aufgabe der Erfindung, ein Verfahren anzugeben, mit dem für die Verdampfung nützliche Parameter in einem Inhalator mit begrenzten Datenverarbeitungsressourcen verlässlich bestimmt werden können.

Die Erfindung löst diese Aufgabe mit den Merkmalen der unabhängigen Patentansprüche.

Erfindungsgemäß ist die elektronische Steuerungsvorrichtung insbesondere nach einem Wechsel einer Verdampferkartusche in dem Inhalator zur Durchführung der folgenden Initialisierungsprozedur eingerichtet: Ausgabe einer Inhalieranforderung an einen Nutzer des Inhalators; bei einem auf die Inhalieranforderung folgenden Zug des Nutzers: Betrieb des Verdampfers mit einer vergleichsweise geringen Verdampfungsleistung, und Aufnehmen einer Zeitmessreihe einer elektrischen Kenngröße des Verdampfers; Ermitteln eines Übergangspunktes zwischen einem Bereich geringer Verdampfung und einem Bereich hoher Verdampfung in der aufgenommenen Zeitmessreihe; Ermitteln und Speichern mindestens eines mit der Initialisierungsprozedur zusammenhängenden Parameters.

Es konnte gezeigt werden, dass der Übergangspunkt bei geringen Heizleistungen deutlich besser detektiert werden kann als bei hohen Heizleistungen, die im Standardbetrieb wünschenswert sind, da sie ein schnelles Ansprechen und eine hohe Dampferzeugung ermöglichen. Die Grundidee ist es daher, zunächst einmalig eine Initialisierungsprozedur für eine Kartusche durchzuführen. Dazu wird der Nutzer, insbesondere nach dem Einlegen eine Verdampferkartusche in den Inhalator, aufgefordert, einen Zug zu nehmen. Dieser Zug wird bei einer vergleichsweise geringen Heizleistung (beispielsweise eingestellt über Pulsweitenmodulation) durchgeführt.

Der Verdampfungs- oder Übergangspunkt ist bei diesen kontrollierten Bedingungen deutlich einfacher zu bestimmen als bei den Standardpuffs mit hoher Leistung. Zudem darf die Berechnung des Übergangspunkts länger dauern, als es im Normal- oder Konsumbetrieb erwünscht ist. Mit anderen Worten ist die Laufzeit der Initialisierungsprozedur weniger kritisch als im normalen Konsumbetrieb. Vorteilhaft kann die Berechnung der (Initialisierungs-)Parameter länger dauern als ein normaler Konsum-Puff durch den Nutzer, insbesondere beispielsweise länger als 1 s.

Erfindungsgemäß beträgt die während der Initialisierungsprozedur eingestellte Heizleistung P maximal 80%, vorteilhaft maximal 60% der im normalen Konsumbetrieb verwendeten mittleren Heizleistung <P> oder maximalen Heizleistung Pmax.

Vorzugsweise wird einer oder mehrere der folgenden Parameter ermittelt und gespeichert: der Wert R0 der elektrische Kenngröße zu Beginn der Zeitmessreihe bei Umgebungstemperatur T0; die Umgebungstemperatur T0; die Zeitdauer tü von einem Anfangspunkt der Zeitmessreihe bis zum Erreichen des Übergangspunktes ÜP; der Wert Rü der elektrischen Kenngröße am Übergangspunkt ÜP. In einer Variante der Initialisierungsprozedur werden vorteilhaft der gemessene Anfangswiderstand R0 (das ist der Widerstand von Verdampfer plus lösbarem Verbinder) bei Umgebungstemperatur T0, die Umgebungstemperatur T0 selbst, die Zeitdauer tü bis zum Erreichen des Übergangspunkts und der Widerstandswert Rü am Übergangspunkt gespeichert.

Die Initialisierungsprozedur kann vorteilhaft über einen Zug (Puff) des Konsumenten durchgeführt werden. Zur Erhöhung der Genauigkeit können aber auch mehrere Züge/Puffs analysiert werden. Im Allgemeinen kann die Initialisierungsprozedur und/oder die Überprüfungsprozedur über einen Zug des Nutzers oder über eine Mehrzahl von Zügen des Nutzers durchgeführt werden.

In einer vorteilhaften Ausführungsform berechnet die Steuerungsvorrichtung ein Maß für die Güte der Ermittlung des Übergangspunktes, und veranlasst bei nicht ausreichender Güte die erneute Durchführung der Initialisierungsprozedur.

Vorzugsweise wird die Heizung des Verdampfers nach Abschluss der Initialisierungsprozedur auf der Grundlage des mindestens einen gespeicherten Parameters geregelt. Der Verdampfer wird also in einer nachfolgenden Konsumphase basierend auf dem nun gespeicherten Modell des Heizers betrieben, d.h. die Regelschwellen werden an die gemessen Daten angepasst.

In einer vorteilhaften Ausführungsform wird die Initialisierungsprozedur sukzessive bei unterschiedlichen Heizleistungen des Verdampfers durchgeführt, um ein genaueres Modell des Heizers erstellen zu können.

Vorzugsweise wird mindestens einer der ermittelten und gespeicherten Parameter nach einem Zug durch den Konsumenten in einer Überprüfungsprozedur bestimmt und mit einem Sollwert verglichen. Dies geschieht insbesondere nach Anschluss der Initialisierungsphase in der Konsumphase. Im Falle von Abweichungen des erneut bestimmten Parameters von dem Sollwert eine vorbestimmte Maßnahme eingeleitet wird, beispielsweise Durchführung einer erneuten Initialisierungsprozedur oder Ausgabe einer Meldung an den Nutzer.

Vorteilhaft werden somit alle oder einige der gespeicherten Parameter (Anfangswiderstand bei Umgebungstemperatur, die Umgebungstemperatur selbst, die Dauer bis zum Erreichen des Übergangspunkts und Widerstandswert am Übergangspunkt) nach einem Zug/Puff in der Konsumphase bestimmt und dem internen Modell bzw. daraus abgeleiteten Sollwerten verglichen. Bei Abweichungen von den erwarteten Sollwerten können entsprechende Maßnahmen eingeleitet werden, wie zum Beispiel erneute Initialisierung, oder Meldung an den Nutzer, etwa in der Form "Kartusche leer", "Anpresskraft und Verschmutzung des Verbinders überprüfen", "unerwarteter Fehler, bitte an Support wenden" und dergleichen. Eine solche Überprüfungsprozedur kann jedes Mal in bestimmten Intervallen oder bei bestimmten Ereignissen (Nutzungsunterbrechung für einen vorbestimmten Zeitraum, Änderung der Außentemperatur um ein vorbestimmtes Maß, Kartuschenwechsel, Einschalten des lnhalators usw.) erfolgen.

In einer vorteilhaften Ausführungsform wird somit die Initialisierungsprozedur und/oder die Überprüfungsprozedur ereignisgesteuert, d.h. nach Feststellung mindestens eines vorbestimmten Ereignisses durchgeführt, beispielsweise Wechsel einer Verdampferkartusche; Einschalten des Inhalators; Nutzungsunterbrechung des Inhalators für einen vorbestimmten Zeitraum; Änderung der Umgebungstemperatur T0 um ein vorbestimmtes Maß.

In einer vorteilhaften Ausführungsform wird bei der Initialisierungsprozedur eine Einschätzung, insbesondere mindestens eine subjektive Empfindung des Inhaliererlebnisses während der Initialisierungsprozedur durch den Konsumenten abgefragt, beispielsweise durch Eingabemittel am Inhalator oder über ein mit dem Inhalator in Kommunikationsverbindung stehendes Mobilkommunikationsgerät. Die abgefragte Einschätzung durch den Konsumenten kann vorteilhaft eine oder mehrere der Kategorien Dampfmenge (beispielsweise zu hoch, zu niedrig, angemessen), Geschmack (beispielsweise zu kratzig, zu mild, angemessen) usw. umfassen. Vorzugsweise kann die abgefragte Einschätzung bei der erfindungsgemäßen Ermittlung des mindestens einen Parameters berücksichtigt werden.

In einer vorteilhaften Ausführungsform wird die Initialisierungsprozedur und/oder die Überprüfungsprozedur zeitgesteuert, d.h. jeweils zu einer bestimmten Uhrzeit oder an einem bestimmten Wochentag, oder periodisch durchgeführt.

Falls die Verdampferkartusche eine auslesbare Kennung aufweist, kann nach Austausch der Verdampferkartusche und Wiedereinsetzen der Verdampferkartusche in den Inhalator vorteilhaft auf das bereits gespeicherte, der Kennung zugeordnete Modell zurückgegriffen werden. Falls eine solche Kennung nicht existiert oder nicht gespeichert ist, wird vorteilhaft bei jedem Kartuschenwechsel eine Initialisierung durchgeführt. Mit anderen Worten kann beim Einsetzen einer Verdampferkartusche mit einer individuellen Kennung und Auslesen der Kennung durch die elektronische Steuerungsvorrichtung mindestens ein dieser Kennung zugeordneter gespeicherter Parameter von der elektronischen Steuerungsvorrichtung abgerufen und zur Verdampfungssteuerung genutzt werden.

Vorzugsweise wird der Übergangspunkt ÜP anhand einer Regression, vorzugsweise einer linearen Regression, an die Zeitmessreihe ermittelt. Es hat sich insbesondere gezeigt, dass der Heizwiderstand bei geringen Heizleistungen sich linear in der Zeit verändert, bis der Übergangspunkt erreicht ist. Daher besteht ein einfaches Verfahren zur Bestimmung des Übergangspunktes darin, eine Gerade ausgehend von t0 an die Messpunkte anzupassen. Der Verdampfungspunkt ist erreicht, wenn der Unterschied zwischen gemessener Kenngröße bzw. gemessenem Widerstand und der Geraden eine voreingestellte Schwelle unter- oder überschreitet.

Ein Verfahren zur Bestimmung des Übergangspunktes kann auch mit Methoden der Künstlichen Intelligenz gefunden werden. Bei dieser Vorgehensweise werden vorher große Datensätze exemplarischer Verdampfungskurven generiert. Nach der Aufnahme kann eine Vorverarbeitung stattfinden. Diese umfasst oftmals Filterung, Überführung in andere Darstellungsweisen wie z.B. Frequenzraum, und/oder einfachere mathematische Operationen wie Integration oder Differenzialrechnung. Die Resultate im neuen und gewöhnlich reduzierten Merkmalsraum werden im Anschluss automatisiert oder manuell abschnittsweise in vorher festgelegte Kategorien klassifiziert. Diese Kategorien können bspw. Heizphase, Verdampfungsphase und Abkühlphase sein. Als Klassifikationsverfahren können beispielsweise künstliche neuronale Netze oder anderweitige Baumstrukturen verwendet werden. Die Erstellung dieser Klassifikatoren kann auch mittels maschinellen Lernens erfolgen. Die generierten Daten bieten z.B. die Möglichkeit die Nutzung des überwachten Lernens. Der fertige Klassifikator kann nach der Lern-/Erstellungsphase direkt in die elektronische Steuerungsvorrichtung einprogrammiert werden. Übergangspunkte können nicht nur direkt durch eine Klasse erkannt, sondern beispielsweise auch durch die Erkennung des Wechsels der detektierten Klasse in eine andere Klasse in quasi Echtzeit ermittelt werden. Solche Systeme sind bekannt für eine relativ hohe Robustheit gegenüber umweltbedingten Schwankungen, wie sie durch Streuung des Widerstandes aufgrund Abweichungen im Produktionsprozess oder der Übergangswiderstände entstehen können. Mit weiterer Analyse und Aufnahme weiterer Messkurven kann die Genauigkeit des Systems stetig verbessert werden. Denkbar wäre auch ein unüberwachtes und damit automatisches Lernen. Die hierfür nötigen Daten könnten dafür direkt vom Inhalator aufgenommen und an zentraler Stelle gesammelt und genutzt werden.

Die Aufgabe wird des Weiteren auch gelöst durch einen Inhalator umfassend einen auf Widerstandsheizung beruhenden Verdampfer und eine elektronische Steuerungsvorrichtung, die zur Durchführung des vorbeschriebenen Verfahrens eingerichtet bzw. programmiert ist.

Die Erfindung wird im Folgenden anhand bevorzugter Ausführungsformen unter Bezugnahme auf die beigefügten Figuren erläutert. Dabei zeigt
- Fig. 1: eine schematische Ansicht eines elektronischen Inhalators;
- Fig. 2: eine schematische Schaltung zur Stromheizung eines Heizelements;
- Fig. 3: ein Widerstands-Zeit-Diagramm mit vier Messreihen für unterschiedliche Heizleistungen;
- Fig. 4A-4D: Widerstands-Zeit-Diagramme mit jeweils einer Messreihe aus Figur 3; und
- Fig. 5: ein 1/tü-Diagramm über der Heizleistung für vier unterschiedliche Heizleistungen.

Der elektronische Inhalator 10, hier ein elektronisches Zigarettenprodukt, umfasst ein Gehäuse 11, in dem ein Luftkanal 30 zwischen mindestens einer Lufteinlassöffnung 31, und einer Luftauslassöffnung 24 an einem Mundende 32 des Zigarettenprodukts 10 vorgesehen ist. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt.

Der Inhalator 10 besteht vorteilhaft aus einem Basisteil 16 und einer auswechselbaren Verdampferkartusche 17, die eine Verdampfungsvorrichtung 20 und einen Flüssigkeitsspeicher 18 umfasst. Die durch die Einlassöffnung 31 angesaugte Luft wird in dem Luftkanal 30 als Luftstrom 34 zu der, durch die, oder an der Verdampfungsvorrichtung 20 entlang geleitet. Die Verdampfungsvorrichtung 20 ist mit dem Flüssigkeitsspeicher 18 verbunden oder verbindbar, in dem mindestens eine Flüssigkeit 33 gespeichert ist. Die Verdampfungsvorrichtung 20 verdampft Flüssigkeit 33, die ihr aus dem Flüssigkeitsspeicher 18 zugeführt wird, und gibt die verdampfte Flüssigkeit als Aerosol/Dampf an einer Auslassseite 26 in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

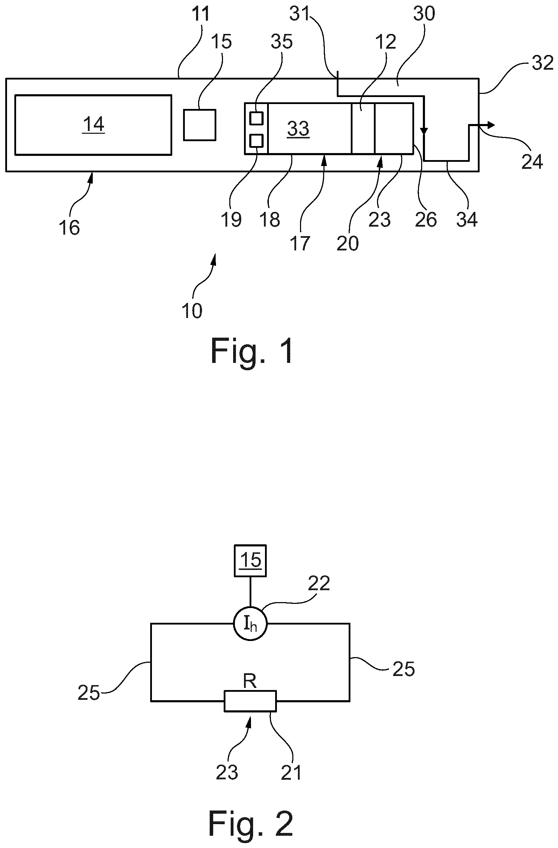

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-lonen-Akku, sein. In dem in Figur 1 gezeigten Beispiel ist der Energiespeicher 14 in einem dem Mundende 32 abgewandten Teil des Inhalators 10 angeordnet. Die Verdampferkartusche 17 ist vorteilhaft zwischen dem Energiespeicher 14 und dem Mundende 32 angeordnet. Die elektronische Steuerungsvorrichtung 15 umfasst mindestens eine digitale Datenverarbeitungseinrichtung, insbesondere einen Mikroprozessor und/oder Microcontroller, in dem Basisteil 16 (wie in Figur 1 gezeigt) und/oder in der Verdampferkartusche 17.

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Inhalators 10 zieht, um zu inhalieren. In diesem Fall steuert die Steuerungsvorrichtung 15 die Verdampfungsvorrichtung 20 an, um Flüssigkeit 33 aus dem Flüssigkeitsspeicher 18 als Aerosol/Dampf in den Luftstrom 34 zuzugeben.

Die in dem Flüssigkeitsspeicher 18 gespeicherte, zu dosierende Flüssigkeit 33 ist beispielsweise eine Mischung umfassend einen oder mehrere der folgenden Bestandteile: 1,2-Propylenglykol, Glycerin, Wasser, mindestens ein Aroma (Flavour), mindestens ein Wirkstoff, beispielsweise Nikotin.

Die Verdampfungsvorrichtung 20 umfasst mindesten einen Verdampfer 23 mit mindestens einem Widerstands-Heizelement 21 (siehe Figur 2) und vorteilhaft ein Dochtelement 12 zum Zuführen von Flüssigkeit 33 aus dem Flüssigkeitsreservoir 18 zu dem Verdampfer 23. Bei in den Inhalator 10 eingesetzter Verdampferkartusche 17 ist das Widerstands-Heizelement 21 mit einer von der elektronischen Steuerungsvorrichtung 15 steuerbaren Heizstromquelle 22 über elektrische Leitungen 25 elektrisch verbunden. Die Heizstromquelle 22 bezieht elektrische Energie aus dem Energiespeicher 14. Aufgrund des Ohmschen Widerstands führt ein Stromfluss durch das elektrisch leitende Heizelement 21 zu einer Erhitzung desselben und daher zu einer Verdampfung von mit dem Heizelement 21 in Kontakt stehender Flüssigkeit. Auf diese Weise erzeugter Dampf/Aerosol entweicht zur Auslassseite 26 aus dem Verdampfer 23 und wird der Luftströmung 34 beigemischt, siehe Figur 1.

Die Verdampfungstemperatur liegt vorzugsweise im Bereich zwischen 100°C und 400 °C, weiter bevorzugt zwischen 150°C und 350 °C, noch weiter bevorzugt zwischen 190 °C und 290 °C.

Die Verdampferkartusche 17 und/oder das Basisteil 16 umfasst vorteilhaft einen digitalen Datenspeicher 35 zum Speichern von die Verdampferkartusche 17 betreffender Information bzw. Parameter. Der Datenspeicher 35 kann Teil der elektronischen Steuerungsvorrichtung 15 oder mit diesem verbunden sein. In dem Datenspeicher 35 ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verdampferkartusche 17, Seriennummer, Herstelldatum und/oder Ablaufdatum, und/oder Zugzahl (Anzahl der Inhalationszüge durch den Konsumenten) bzw. der Nutzungszeit gespeichert.

Die Verdampfungsvorrichtung 20 kann vorteilhaft eine Messschaltung 19 zur Bestimmung der Temperatur des Heizelements 21 durch Widerstandsmessung des Heizelements 21 aufweisen.

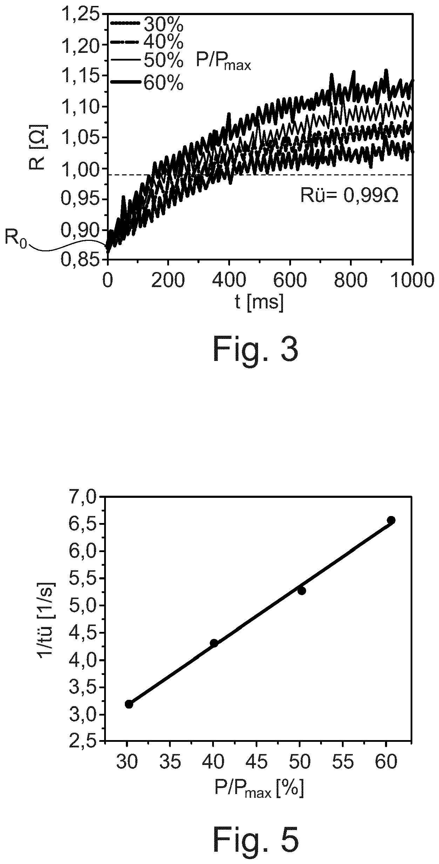

Die Diagramme in den Figuren 3 bis 5 illustrieren eine erfindungsgemäße Initialisierungsprozedur.

Eine Initialisierungsprozedur wird von der Steuerungsvorrichtung 15 automatisch gestartet, wenn eine oder mehrere vorbestimmte Initialisierungsbedingungen erfüllt und von der Steuerungsvorrichtung 15 festgestellt werden. Eine solche Initialisierungsbedingung ist insbesondere ein Kartuschenwechsel, d.h. eine Initialisierungsprozedur wird vorteilhaft nach dem Einsetzen einer Verdampferkartusche 17 in den Inhalator 10 gestartet. Die Steuerungsvorrichtung 15 ist zur Detektion eines solchen Kartuschenwechsels eingerichtet. Dies kann beispielsweise über eine Detektionsschaltung erfolgen, die das Vorhandensein oder Nichtvorhandensein einer Verdampferkartusche 17 in einer entsprechenden Aufnahme des Inhalators 10 fortlaufen überwacht und mittels eines digitalen Werts (Flag) markiert. Eine Änderung des digitalen Werts (des Flags) kann beispielsweise als Interrupt für die Software in der Steuerungsvorrichtung 15 verwendet werden, sodass vorteilhaft jederzeit, etwa auch im Standby, überwacht werden kann, ob eine Verdampferkartusche 17 in den Inhalator 10 eingesetzt ist oder nicht. Ein Kartuschenwechsel kann somit vorteilhaft in allen Betriebszuständen des Inhalators 10 detektiert werden.

Zusätzlich oder alternativ kann eine Initialisierungsprozedur bei der Feststellung anderer Ereignisse, beispielsweise Einschalten des Inhalators 10, und/oder zeitgesteuert gestartet werden.

Wenn die Steuerungsvorrichtung 15 eine Initialisierungsbedingung, beispielsweise das Einsetzen einer Verdampferkartusche 17 in den Inhalator feststellt, wird die Initialisierungsprozedur gestartet und zu diesem Zweck an den Konsumenten bzw. Nutzer eine Inhalieranforderung ausgegeben. Dies kann über eine optische, akustische und/oder haptische Anzeigeeinrichtung des Inhalators 10 geschehen. Zusätzlich oder alternativ kann die Inhalieranforderung über ein externes elektronisches Gerät, das mit dem Inhalator 10 drahtlos oder mittels Kabel verbunden ist, an den Nutzer ausgegeben werden.

Wenn die Steuerungsvorrichtung 15 mittels des bereits erwähnten Sensors (Drucksensor, Druck- oder Strömungsschalter) feststellt, dass der Nutzer der Inhalieranforderung nachkommt und an dem Mundende des Inhalators 10 zieht, wird der Heizwiderstand 21 des Verdampfers 23 über den (Initialisierungs-)Zug des Konsumenten mit einer Heizleistung P betrieben, die wesentlich geringer ist als die maximale Leistung Pmax im Normal- oder Konsumbetrieb. Die Heizleistung P wird beispielsweise über Pulsweitenmodulation von der Steuerungseinrichtung 15 in der Heizstromquelle 22 eingestellt.

Im Ausführungsbeispiel gemäß den Figuren 3 bis 5 werden vier Messungen entsprechend P/Pmax = 30%, 40%, 50% und 60% betrachtet, wobei jede Messung mindestens einem (Initialisierungs-) Zug durch den Konsumenten entspricht. Die Initialisierungsprozedur kann auf der Grundlage eines oder mehrerer Züge bei einer bestimmten Heizleistung (beispielsweise bei P/Pmax = 40%) erfolgen oder eine Mehrzahl von Messungen über jeweils einen oder mehrere Züge bei unterschiedlichen Heizleistungen umfassen, wodurch die Genauigkeit der Initialisierungsprozedur ggf. gesteigert werden kann. Die in der Initialisierungsphase eingestellte geringe Heizleistung P erzeugt typischerweise eine für Genuss bzw. Wirkung zu geringe Dampfmenge bzw. zu geringen Wirkstoffgehalt im Dampf. Die Initialisierungsphase liegt zeitlich vor dem Beginn der eigentlichen Konsumphase.

In Figur 3 ist der (temperaturabhängige) Widerstand R des Heizwiderstands 21 in Ohm gegenüber der Zeit in ms aufgetragen. Die Anfangszeit t = 0 ms entspricht dem Beginn der Heizphase mit niedriger Heizleistung P. Gezeigt sind die Widerstandskurven R(t) für die erste Sekunde (1000 ms) eines Initialisierungspuffs mit einer relativen Leistung P/Pmax (bzw. Pulsweitenverhältnis) von 30%, 40%, 50% und 60%. Das überlagerte periodische Signal wird hier durch Pulsweitenmodulation verursacht. Als dicke Linien sind die gleitenden Durchschnitte über jeweils n Messpunkte (beispielsweise n=31) aufgetragen.

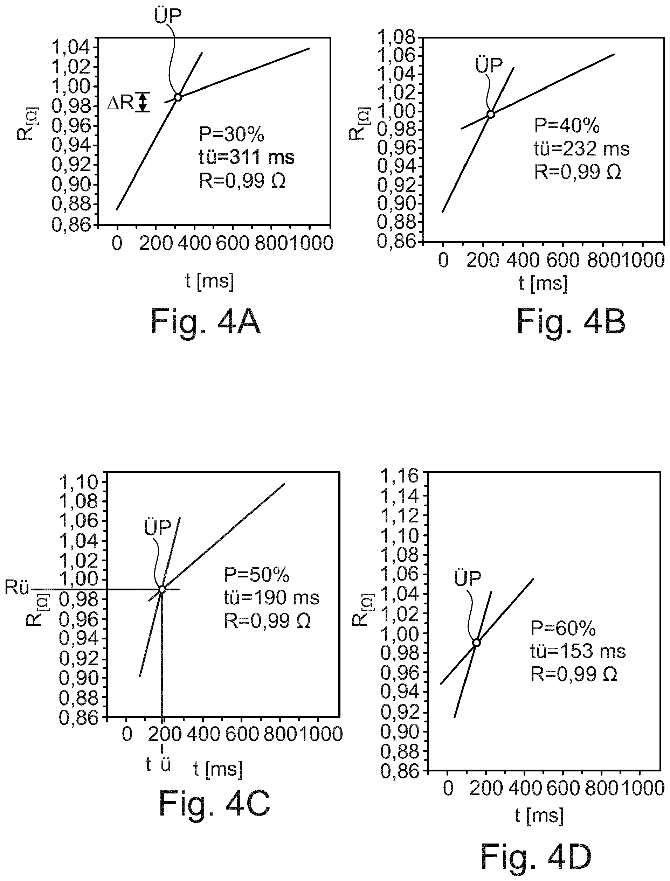

In den Abbildungen 4A bis 4D sind die vier gemittelten Kurven (dicke Linien in Figur 3) der besseren Übersichtlichkeit wegen nochmals separat gezeigt.

Wie aus den Figuren 3, 4A-4D ersichtlich ist, steigt der temperaturabhängige Widerstand ausgehend von t = 0 ms zunächst etwa linear mit einer relativ großen Steigung an, was auf einer schnellen Aufheizung des Heizelements 21 beruht. In einem späteren Zeitbereich ab 200 ms bis 300 ms steigt der Widerstand weiter an, jedoch mit einer wesentlich kleineren Steigung, was auf einer wesentlich langsameren Aufheizung des Heizelements 21 beruht, weil ab einem gewissen Punkt (Übergangspunkt) erhebliche Verdampfung einsetzt und die Verdampfungsenergie nicht zur Aufheizung des Heizelements 21 zur Verfügung steht.

Der Übergangspunkt ÜP (oder Verdampfungspunkt) zwischen dem Bereich geringer Verdampfung und dem Bereich hoher Verdampfung (siehe Fig. 4A-4D) stellt einen nützlichen Parameter für die Verdampfungsregelung im normalen Konsumbetrieb des Inhalators 10 dar. Die Steuerungsvorrichtung 25 ist daher vorteilhaft zur rechnerischen Ermittlung des Übergangspunktes ÜP aus der oder den Widerstands-Zeit-Messreihen eingerichtet.

In einer Ausführungsform wird der Übergangspunkt ÜP als Schnitt punkt zweier Geraden ermittelt, wie in Fig. 4A-4D gezeigt. Die ersten Gerade wird an die Messreihe in einem Bereich ausgehend von t0= 0 ms bis beispielsweise t1 = 100 ms oder 200 ms so angepasst bzw. angefittet, dass die Abweichung von der Messkurve oder der geglätteten Messkurve minimal ist (Bereich geringer Verdampfung), z.B. an die ersten 50 Messpunkte im Abstand von je 2 ms. Die zweite Gerade wird an die Messreihe in einem Bereich ausgehend von t2 > t1 bis beispielsweise t3 = 600 ms so angepasst bzw. angefittet, dass die Abweichung von der Messkurve oder der geglätteten Messkurve wiederum minimal ist (Bereich hoher Verdampfung). Es ergibt sich in allen Fällen, d.h. unabhängig von der jeweiligen Heizleistung P, ein Übergangswiderstand (y-Wert des Übergangspunkts in den Widerstands-Zeit-Diagrammen) von Rü = 0,99 Ω. Die Aufheizdauer oder Übergangszeit tü (y-Wert des Übergangspunkts in den Widerstands-Zeit-Diagrammen) ist dagegen je nach Heizleistung unterschiedlich, weil eine höhere Heizleistung eine schnellerer Aufheizung und ein früheres Erreichen des Übergangspunktes bedeutet (beispielsweise P/Pmax = 30%, tü = 311 ms; P/Pmax = 60%, tü = 153 ms).

Andere Verfahren zur Bestimmung des Übergangspunktes aus der oder den Messreihen sind möglich. Beispielsweise könnte es ausreichen, nur eine Gerade an die Messreihe in einem Bereich ausgehend von t0= 0 ms bis beispielsweise t1 = 100 ms oder 200 ms so anzupassen bzw. anzufitten, dass die Abweichung von der Messkurve oder der geglätteten Messkurve minimal ist (Bereich geringer Verdampfung). Der Übergangspunkt ÜP ist erreicht, wenn der Unterschied ΔR zwischen der Geraden und dem entsprechenden Widerstands-Messwert (bzw. der Messkurve oder Messreihe) einen voreingestellte Schwellwert Rt überschreitet, siehe Figur 4A.

Weitere Verfahren zur Bestimmung des Übergangspunktes ÜP sind aus der DE 10 2019 113 645.8 bekannt, deren Offenbarung insoweit in die vorliegende Anmeldung aufgenommen wird. Auch die Ermittlung des Übergangspunkts ÜP mittels eines mit Methoden der künstlichen Intelligenz gewonnenen Algorithmus ist möglich, wie dies zuvor bereits erläutert wurde.

In dem in Figur 5 gezeigten Diagramm sind die aus den Figuren 4A-4D entnommen inversen Aufheizdauern 1/tü über der Heizleistung P (hier relativ zu Pmax) aufgetragen. Die inversen Aufheizdauern 1/tü liegen auf einer durch den Ursprung verlaufenden Geraden, weil die aufgebrachte Heizenergie bis zum Erreichen des Übergangspunkts ÜP in allen Fällen (unabhängig von der Heizleistung P) gleich ist.

Der Verdampfer 23 bzw. das Heizelement 21 können durch folgende Parameter beschrieben werden: Rü, hier beispielsweise 0,99 Ω; R0(T0), wobei T0 die Umgebungstemperatur zum Anfangszeitpunkt t0 = 0 ms ist; hier beispielsweise R0(T0) = 0,88 Ω, siehe Figur 3; und die Steigung der Kurve im Diagramm 1/tü vs. P, siehe Figur 5. Dieser Parametersatz Rü, R0(T0), 1/tü-Steigung kann als Modell des Heizers bzw. Verdampfers 23, kurz Heizermodell, bezeichnet werden.

Grundsätzlich reicht eine Messung bei einer bestimmten Heizleistung aus, um den Übergangspunkt ÜP bzw. sämtliche Parameter des Heizermodells zu bestimmen. Die Durchführung von mehreren Messungen bei gleicher Heizleistung und/oder unterschiedlichen Heizleistungen erhöht jedoch die Zuverlässigkeit und Genauigkeit der Messung.

Das Heizermodell (Rü, R0(T0), 1ltü-Steigung) oder einzelne Parameter desselben sind nützliche Parameter für die Verdampfungsregelung in einer auf die Initialisierung folgende Konsumphase. Diese Regelung kann beispielhaft erfolgen wie in der DE 10 2019 113 645.8 beschrieben, deren Offenbarung betreffend die Stromregelung des Verdampfers 23 in die vorliegende Anmeldung aufgenommen wird. Im einfachsten Fall kann der Stromwert Iv der referenzierten Anmeldung aus dem Übergangswiderstand Rü bestimmt werden: Iv = Rü / U, wobei U die bekannte Heizspannung ist. Sodann kann eine Regelung des Heizstroms Ih in einem Bereich I1 < Iv < I2 erfolgen. Andere Verdampfungsregelungen auf der Grundlage eines oder mehrerer Parameter des Heizermodells, und/oder eines oder mehrerer davon abgeleiterer Parameter wie tü, t0, Temperatur am Übergangspunkt Tü etc. sind möglich.

Nach dem zuvor Gesagten überwacht daher die Steuerungseinrichtung 15 einen oder mehrere Parameter während der folgenden Züge (Puffs) in der Konsumphase und leitet bei (von entsprechenden gespeicherten Sollwerten) erheblich abweichenden Werten geeignete Maßnahmen ein.

## Patentansprüche

1. Verfahren zum Bestimmen mindestens eines Parameters für die Verdampfung in einem Inhalator (10) umfassend einen auf Widerstandsheizung beruhenden Verdampfer (23) und eine elektronische Steuerungsvorrichtung (15), wobei die elektronische Steuerungsvorrichtung (15), insbesondere nach einem Wechsel einer Verdampferkartusche (17) in dem Inhalator (10), zur Durchführung der folgenden Initialisierungsprozedur eingerichtet ist:
- Ausgabe einer Inhalieranforderung an einen Nutzer des Inhalators (10);
- bei einem auf die Inhalieranforderung folgenden Zug des Nutzers:
- - Betrieb des Verdampfers (23) mit einer vergleichsweise geringen Verdampfungsleistung P, und
- - Aufnehmen einer Zeitmessreihe einer elektrischen Kenngröße des Verdampfers (23);
- Ermitteln eines Übergangspunktes ÜP zwischen einem Bereich geringer Verdampfung und einem Bereich hoher Verdampfung in der aufgenommenen Zeitmessreihe;
- Ermitteln und Speichern mindestens eines mit der Initialisierungsprozedur zusammenhängenden Parameters **dadurch gekennzeichnet, dass** die während der Initialisierunqsprozedur eingestellte Heizleistung P maximal 80% der im normalen Konsumbetrieb verwendeten mittleren Heizleistung <P> oder der maximalen Heizleistung Pmax beträgt.

2. Verfahren nach Anspruch 1, **dadurch gekennzeichnet, dass** einer oder mehrere der folgenden Parameter ermittelt und gespeichert wird:
- der Wert R0 der elektrische Kenngröße zu Beginn der Zeitmessreihe bei Umgebungstemperatur T0;
- die Umgebungstemperatur T0;
- die Zeitdauer tü von einem Anfangspunkt der Zeitmessreihe bis zum Erreichen des Übergangspunktes ÜP;
- der Wert Rü der elektrischen Kenngröße am Übergangspunkt ÜP.

3. Verfahren nach einem der vorangehenden Ansprüche, **dadurch gekennzeichnet, dass** die elektronische Steuerungsvorrichtung (15) ein Maß für die Güte der Ermittlung des Übergangspunktes ÜP berechnet und bei nicht ausreichender Güte die erneute Durchführung der Initialisierungsprozedur veranlasst.

4. Verfahren nach einem der vorangehenden Ansprüche, **dadurch gekennzeichnet, dass** die Heizung des Verdampfers (23) nach Abschluss der Initialisierungsprozedur auf der Grundlage des mindestens einen gespeicherten Parameters geregelt wird.

5. Verfahren nach einem der vorangehenden Ansprüche, **dadurch gekennzeichnet, dass** die Initialisierungsprozedur sukzessive bei unterschiedlichen Heizleistungen des Verdampfers (23) durchgeführt wird.

6. Verfahren nach einem der vorangehenden Ansprüche, **dadurch gekennzeichnet, dass** mindestens einer der ermittelten und gespeicherten Parameter nach einem Zug durch den Konsumenten in einer Überprüfungsprozedur bestimmt und mit einem Sollwert verglichen wird.

7. Verfahren nach Anspruch 6, **dadurch gekennzeichnet, dass** im Falle von Abweichungen des erneut bestimmten Parameters von dem Sollwert eine vorbestimmte Maßnahme eingeleitet wird, beispielsweise Durchführung einer erneuten Initialisierungsprozedur oder Ausgabe einer Meldung an den Nutzer.

8. Verfahren nach einem der vorangehenden Ansprüche, **dadurch gekennzeichnet, dass** die Initialisierungsprozedur und/oder die Überprüfungsprozedur nach Feststellung mindestens eines vorbestimmten Ereignisses durchgeführt wird, beispielsweise
- Wechsel einer Verdampferkartusche (17);
- Einschalten des Inhalators (10);
- Nutzungsunterbrechung des Inhalators (10) für einen vorbestimmten Zeitraum;
- Änderung der Umgebungstemperatur T0 um ein vorbestimmtes Maß.

9. Verfahren nach einem der vorangehenden Ansprüche, **dadurch gekennzeichnet, dass** bei der Initialisierungsprozedur eine Einschätzung durch den Konsumenten abgefragt und vorteilhaft bei der Ermittlung des mindestens einen Parameters berücksichtigt wird.

10. Verfahren nach einem der vorangehenden Ansprüche, **dadurch gekennzeichnet, dass** die Initialisierungsprozedur und/oder die Überprüfungsprozedur
- zeitgesteuert oder periodisch, und/oder
- über einen Zug des Nutzers oder über eine Mehrzahl von Zügen des Nutzers
durchgeführt wird.

11. Verfahren nach einem der vorangehenden Ansprüche, **dadurch gekennzeichnet, dass** beim Einsetzen einer Verdampferkartusche (17) mit einer individuellen Kennung und Auslesen der Kennung durch die elektronische Steuerungsvorrichtung (15) mindestens ein dieser Kennung zugeordneter gespeicherter Parameter von der elektronischen Steuerungsvorrichtung (15) abgerufen wird.

12. Verfahren nach einem der vorangehenden Ansprüche, **dadurch gekennzeichnet, dass** der Übergangspunkt ÜP anhand einer Regression, vorzugsweise einer linearen Regression, an die Zeitmessreihe ermittelt wird.

13. Inhalator (10) umfassend einen auf Widerstandsheizung beruhenden Verdampfer (23) und eine elektronische Steuerungsvorrichtung (15), **dadurch gekennzeichnet, dass** die elektronische Steuerungsvorrichtung (15) zur Durchführung des Verfahrens nach einem der vorangehenden Ansprüche eingerichtet ist.

## Claims

1. Method for determining at least one parameter for vaporization in an inhaler (10) comprising a vaporizer (23), based upon resistance heating, and an electronic control device (15), wherein the electronic control device (15) is configured to carry out the following initialization procedure - in particular, after a replacement of a vaporizer cartridge (17) in the inhaler (10):
- outputting an inhalation request to a user of the inhaler (10);
- in the case of a puff taken by the user following the inhalation request:
- - operating the vaporizer (23) with a comparatively low vaporization capacity P, and
- - recording a time measurement series of an electrical parameter of the vaporizer (23);
- determining a transition point ÜP between a region of low vaporization and a region of high vaporization in the recorded time measurement series;
- determining and storing at least one parameter associated with the initialization procedure,
**characterized in that** the heating power P set during the initialization procedure is not more than 80% of the average heating power <P> or the maximum heating power Pmax used in normal consumption operation.

2. Method according to claim 1, **characterized in that** one or more of the following parameters are determined and stored:
- the value R0 of the electrical parameter at the beginning of the time measurement series at ambient temperature T0;
- the ambient temperature T0;
- the time period tü from an initial point of the time measurement series until the transition point ÜP is reached;
- the value Rü of the electrical parameter at the transition point ÜP.

3. Method according to one of the preceding claims, **characterized in that** the electronic control device (15) calculates a measure of the quality of the determination of the transition point ÜP, and, in case of insufficient quality, causes the initialization procedure to be carried out again.

4. Method according to one of the preceding claims, **characterized in that** the heater of the vaporizer (23) is controlled on the basis of the at least one stored parameter after completion of the initialization procedure.

5. Method according to one of the preceding claims, **characterized in that** the initialization procedure is carried out successively at different heating powers of the vaporizer (23).

6. Method according to one of the preceding claims, **characterized in that** at least one of the determined and stored parameters is determined after a consumer puff in a checking procedure and is compared with a target value.

7. Method according to claim 6, **characterized in that**, in the case of deviations of the redetermined parameter from the target value, a predetermined measure is initiated - for example, performing a re-initialization procedure or outputting a message to the user.

8. Method according to one of the preceding claims, **characterized in that** the initialization procedure and/or the checking procedure is carried out after determination of at least one predetermined event, for example
- replacement of a vaporizer cartridge (17);
- switching on of the inhaler (10);
- interruption of use of the inhaler (10) for a predetermined period of time;
- change in ambient temperature T0 by a predetermined value.

9. Method according to one of the preceding claims, **characterized in that** an assessment by the consumer is requested in the initialization procedure and is, advantageously, taken into account when determining the at least one parameter.

10. Method according to one of the preceding claims, **characterized in that** the initialization procedure and/or the checking procedure is carried out
- in a time-controlled manner or periodically, and/or
- via one user puff or via a plurality of user puffs.

11. Method according to one of the preceding claims, **characterized in that**, when inserting a vaporizer cartridge (17) with an individual identifier and having the identifier read out by the electronic control device (15), at least one stored parameter assigned to this identifier can be accessed by the electronic control device (15).

12. Method according to one of the preceding claims, **characterized in that** the transition point ÜP is determined based upon a regression, preferably a linear regression, to the time measurement series.

13. Inhaler (10) comprising a vaporizer (23), based upon resistance heating, and an electronic control device (15), **characterized in that** the electronic control device (15) is configured to carry out the method according to one of the preceding claims.

## Revendications

1. Procédé pour déterminer au moins un paramètre de vaporisation dans un inhalateur (10) comprenant un vaporisateur (23), basé sur un chauffage par résistance, et un dispositif de commande électronique (15), dans lequel le dispositif de commande électronique (15), en particulier après un remplacement d'une cartouche de vaporisateur (17) dans l'inhalateur (10), est configuré pour exécuter la procédure d'initialisation suivante :
- émettre une demande d'inhalation destinée à un utilisateur de l'inhalateur (10) ;
- lors d'une bouffée de l'utilisateur qui suit la demande d'inhalation :
-- faire fonctionner le vaporisateur (23) avec une puissance de vaporisation P comparativement faible, et
-- enregistrer une série chronologique de mesures d'une caractéristique électrique du vaporisateur (23) ;
- déterminer un point de transition ÜP entre une zone de faible vaporisation et une zone de forte vaporisation dans la série chronologique de mesures enregistrée ;
- déterminer et mémoriser au moins un paramètre associé à la procédure d'initialisation, **caractérisé en ce que** la puissance de chauffage P réglée pendant la procédure d'initialisation est au plus égale à 80 % de la puissance calorifique moyenne <P> utilisée en mode de consommation normale ou de la puissance calorifique maximale Pmax.

2. Procédé selon la revendication 1, **caractérisé en ce qu'**un ou plusieurs des paramètres suivants sont déterminés et mémorisés :
- la valeur R0 de la caractéristique électrique au début de la série chronologique de mesures à température ambiante T0 ;
- la température ambiante T0 ;
- la durée tü du point de départ de la série chronologique de mesures jusqu'à ce que le point de transition ÜP soit atteint ;
- la valeur Rü de la caractéristique électrique au point de transition ÜP.

3. Procédé selon l'une des revendications précédentes, **caractérisé en ce que** le dispositif de commande électronique (15) calcule une mesure d'une qualité de la détermination du point de transition ÜP et, si la qualité n'est pas suffisante, entraîne une nouvelle exécution de la procédure d'initialisation.

4. Procédé selon l'une des revendications précédentes, **caractérisé en ce que** le chauffage du vaporisateur (23) est régulé après la fin de la procédure d'initialisation sur la base du au moins un paramètre mémorisé.

5. Procédé selon l'une des revendications précédentes, **caractérisé en ce que** la procédure d'initialisation est exécutée successivement à différentes puissances de chauffage du vaporisateur (23).

6. Procédé selon l'une des revendications précédentes, **caractérisé en ce qu'**au moins un des paramètres déterminés et mémorisés est déterminé après une bouffée par le consommateur pendant une procédure de contrôle et est comparé à une valeur de consigne.

7. Procédé selon la revendication 6, **caractérisé en ce que**, en cas d'écarts du paramètre de nouveau déterminé par rapport à la valeur de consigne, une mesure prédéterminée est déclenchée, par exemple en exécutant une nouvelle procédure d'initialisation ou en envoyant un message à l'utilisateur.

8. Procédé selon l'une des revendications précédentes, **caractérisé en ce que** la procédure d'initialisation et/ou la procédure de contrôle est mise en oeuvre après l'observation d'au moins un événement prédéterminé, par exemple :
- le remplacement d'une cartouche de vaporisateur (17) ;
- la mise en marche de l'inhalateur (10) ;
- l'interruption d'utilisation de l'inhalateur (10) pendant une période de temps prédéterminée ;
- la variation de la température ambiante T0 d'une grandeur prédéterminée.

9. Procédé selon l'une des revendications précédentes, **caractérisé en ce que** pendant la procédure d'initialisation, une estimation est demandée par le consommateur et est avantageusement prise en compte lors de la détermination du au moins un paramètre.

10. Procédé selon l'une des revendications précédentes, **caractérisé en ce que** la procédure d'initialisation et/ou la procédure de contrôle est mise en oeuvre
- de façon commandée dans le temps ou périodique, et/ou
- sur une bouffée de l'utilisateur ou sur une pluralité de bouffées de l'utilisateur.

11. Procédé selon l'une des revendications précédentes, **caractérisé en ce que**, lors de l'insertion d'une cartouche de vaporisateur (17) ayant un identifiant individuel et de la lecture de l'identifiant par le dispositif de commande électronique (15), au moins un paramètre mémorisé associé à cet identifiant est récupéré par le dispositif de commande électronique (15).

12. Procédé selon l'une des revendications précédentes, **caractérisé en ce que** le point de transition ÜP est déterminé par une régression, de préférence une régression linéaire, sur la série chronologique de mesures.

13. Inhalateur (10) comprenant un vaporisateur (23), basé sur un chauffage par résistance, et un dispositif de commande électronique (15), **caractérisé en ce que** le dispositif de commande électronique (15) est configuré pour mettre en oeuvre le procédé selon l'une des revendications précédentes.
